Europäisches Patentamt

European Patent Office

Office européen des brevets

(11) Publication number: **0 312 953**
**A2**

## EUROPEAN PATENT APPLICATION

(21) Application number: **88117231.6**

(22) Date of filing: **17.10.88**

(51) Int. Cl.⁴: **A61H 33/00**

(30) Priority: **19.10.87 IT 2232787**

(43) Date of publication of application:
**26.04.89 Bulletin 89/17**

(84) Designated Contracting States:
**DE ES FR GB SE**

(71) Applicant: **Jacuzzi Europe Spa**
**S.S. Pontebbana km 97,2**
**I-33098 Valvasone (Pordenone)(IT)**

(72) Inventor: **Soissons, Emanuele Villafranca**
**Via Martiri della Libertà, 10**
**I-33098 Valvasone (Pordenone)(IT)**

(74) Representative: **Dragotti, Gianfranco et al**
**SAIC BREVETTI s.r.l. Viale Bianca Maria, 15**
**I-20122 Milano(IT)**

(54) Disinfection assembly for the hydraulic circuit of a hydromassage system.

(57) An assembly for the disinfection of the hydraulic circuit (10,10a) of a hydromassage system comprising a tub (12), at least a suction fitting (20, 74) and at least a delivery fitting (30a-f) in which a disinfection liquid from a source (40) in said circuit (10,10a), the suction and delivery fittings of which are maintained in the closed condition, entirely fills said circuit (10, 10a) and comes slowly out towards a tub outlet through a duct of reduced diameter by which the outflow of said disinfection liquid is slowed down, permitting the permanence thereof in the said circuit (10, 10a) even after the introduction of the liquid is completed, for a period sufficient to permit said disinfection.

Fig.1

## Disinfection assembly for the hydraulic circuit of a hydromassage system.

The present invention relates to a disinfection assembly for hydraulic circuits of hydromassage systems, applicable to bath tubs or the like. The hydromassage practice consisting in having jets of water under pressure admixed with air impinging against the body of an user immersed within a tub or pool filled with water, is well known and is becoming and more more widespread.

Owing to the fact, that for reasons of water saving, said jets are obtained by using the same water of the tub or pool which is circulated within a hydraulic circuit comprising a recirculation pump, at the very end said water in which the use body is immersed is loaded with particules and substances coming from the body itself.

Most of said particles and substances are eliminated by discharging the water from the tub or pool, but a minor amount thereof may remain in a residual water body which remains within the said hydraulic circuit after the use.

Owing to the fact said particles and substances in water may constitute a culture broth for several microorganisms which are naturally present everywhere, the water stagnation in said hydraulic circuit may lead to the multiplication of the same microorganisms with hygienic consequences with are definitely objectionable.

On this fact depends the necessity of periodically disinfecting the hydraulic circuit of the said hydromassage systems.

Said disinfection can be easily carried out be introducing a certain amount of disinfectant within the water of the tub associated to the hydromassage system, having it operated in the absence of a user within the tub , for a period sufficient to have the disinfecting substances entering the hydraulic circuit and having them remaining therein to obtain the desired disinfection effect.

This method can also be sufficiently effective, but requires the use of a great amount of disinfecting substances in order to have an effective concentration thereof in the water body of the tub and the intervention of personnel to take care of the carrying out of said disinfection.

The purpose of the present invention is that of providing an assembly for the disinfection of the hydraulic circuit of hydromassage systems in semiautomatic or totally automatic manner, by using a minimum amount of disinfecting substances and requiring a minimum or null intervention of attendant personnel.

Another purpose of the present invention is that of providing an assembly for disinfection of the hydraulic circuit of the hydromassage systems using means controlled by a control station or programmer of said system associated to a tub containing it, which may be actuated at will by an user or automatically after a cycle of use of said tub.

These and other purposes are achieved by providing a disinfection assembly of the hydraulic circuit (10, 10a) of a hydromassage system for bath tubs (12) of the type comprising a circulation pump (24), at least a suction fitting (20,74) with can be closed, said tub (12) further comprising a conventional outlet (14, 14a), charactized in that said hydraulic circuit (10, 10a) is connected through at least a controllable valve (44) to a source of disinfecting liquid (40), and in that said hydraulic circuit (10,10a) is connected at its lowest point to the outlet (14, 14a) of the tub (12) through a duct (52, 106) of diameter definitely lesser than that of the duct forming said hydraulic circuit (10,10a), in said duct (52, 106) being mounted a check or non return valve which can be opened under the action of a liquid column contained in said hydraulic circuit so as permit the outflow of said liquid within a predetermined time such as to permit a sufficient disinfecting action in said hydraulic circuit.

Furthermore, the outlet (14a) of the tub (12) comprises a suction fitting (74) for the hydromassage system, provided with an on-off valve (98) permitting the passage of liquid only from said outlet (14a) towards said system (10a) positioned upstream of said means (80) for the closure of said outlet and a duct (106) provided with a throttling section (108) and with a non return valve (110), which can be opened under the action of a liquid column contained in said hydraulic circuit , and connnecting a part of the suction fitting (22) downstream of the related on-off valve (98) with a part (72) downstream of said closing means (80) of said outlet. Still furthermore, said on-off valve (98) on the suction fitting (14a) is a non return valve which is maintained in the closed condition by elastic means and is opened by the operation of the pump (24) of the hydromassage system generating a depression in said suction fitting (22).

Preferably, said on-off valve (98) on the suction fitting is an electrovalve actuated by electromagnet (102) connected to the pump (24) of the hydromassage system.

Still preferably, said duct (106) connecting the suction pipe downstream of the related check valve with the outlet (72) downstream of its closing means (80) contains a valve (110) provided with an adjustable spring (122) to control the outflow time of the liquid from said hydromassage system.

According to a first embodiment of the present invention the said source of disinfecting liquid (40) consists of a tank containing a disinfecting solution.

According to a another embodiment of the present invention, the said source of disinfecting liquid (40) consists of a disinfectant delivery device, connected to the pressure tap water system (42) comprising a controllable valve (44), a stored amount of disinfecting substance and a dosing device (46) by which the disinfecting solution is fed into said hydromassage system.

The feature and advantages of the present invention shall be evident from the following detailed description of two embodiments, taken into consideration with the enclosed drawings in which:

- figure 1 is a schematic view of a first and simpler embodiment of the present invention;

- figure 2 is a schematic view of a second and more preferred embodiment of the present invention;

figure 3 is a cross-section view of the particular combination of outlet and suction fitting used in the embodiment of figure 2.

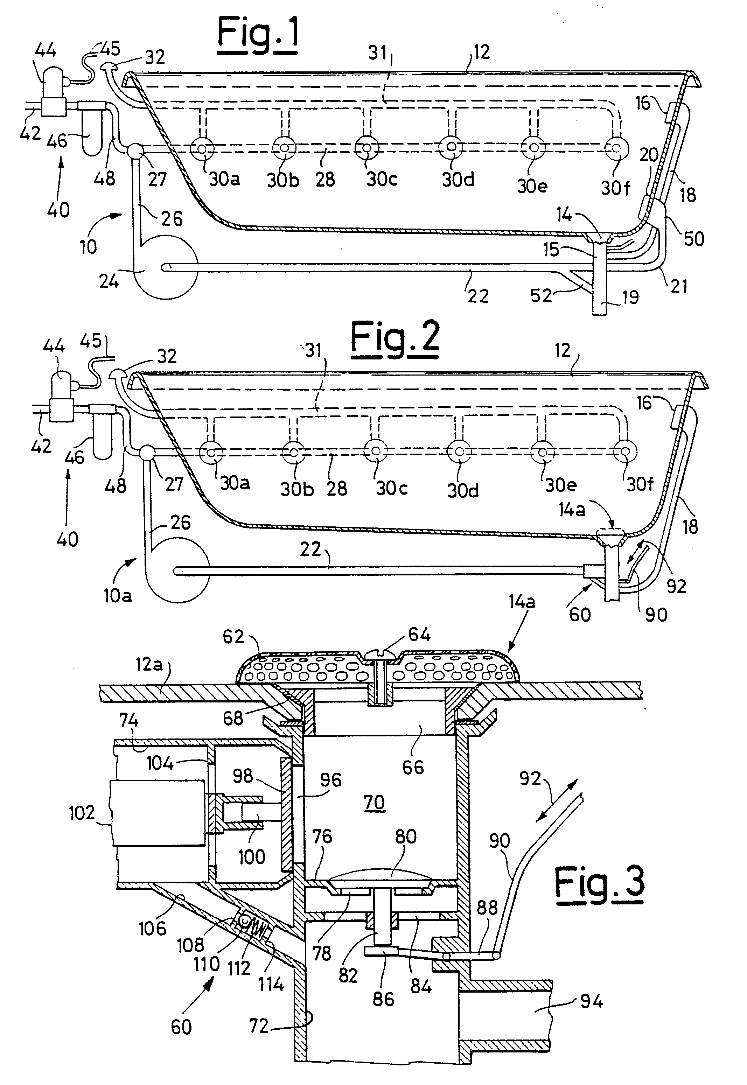

Referring now to figure 1, the first embodiment of the present invention comprises a hydraulic circuit 10 of a hydromassage system and a bath tub 12, said tub being conventionally provided with a discharge fitting (14) which can be closed either by means of a simple plug or by means of a ratchet device 15 which can be remotely actuated out of the tub, with an overflow outlet (16) connected by means of a duct 18 to a next duct 19 of discharge from the tub.

Near the bottom of the tub 12 at least one suction fitting 20 is provided, connected by means of its duct 21 to a suction pipe 22 of a circulation pump 24, which is part of the hydromassage system 10. To said circulation pump 24 a delivery pipe 26 is connected which in turn joins a header 27 extended by a delivery duct 28 conveying water to a number of delivery fittings 30a- 30f, to each of which also an air duct 31 s connected by which air from the external atmosphere is taken through a fitting 32.

To the hydromassage system 10, particularly to the header 27 a disinfectant delivery system 40 is connected consisting of a duct 42 connected to the tap water system , a controlled valve such as the electrovalve 44 controlled through a cable 45 from a hydromassage programmer or like control systems, a water filter 46 provided with a reserve of concentrated disinfectant which can be fed into through a dosing device (not shown) to a duct 48 connected to the header 27 on the delivery pipe 26 of the pump 24.

To complete the disinfection assembly a bottom non-return valve 50 is provided within the suction fitting 20 together with throttling means in a duct 52, connecting said suction pipe 22 with said outlet duct 19, provided with a normally open valve which is closed when the action of the pump 24 generates a depression in the suction pipe 22.

Figures 2 and 3 shall be now taken into consideration, respectively showing the second embodiment of the present invention and a combination of outlet and the suction fitting particularly preferred in this second embodiment.

The hydraulic circuit 10a of said second embodiment is similar to that of the first embodiment apart from that, instead of having a suction fitting 20 separated from the tub outlet 14, it comprises a combination 60 fulfilling the function of said outlet 14 and of said suction fitting 20, placed under an outlet 14a provided with means for intercepting solid bodies, such as a perforated plate 62 secured into position by suitable means, such as a screw 64, to a discharge fitting 66 placed in an outlet opening 68 present in the bottom 12a of the tub.

The said discharge fitting 66 has connected thereto (for example screwed thereto) a connection defining a chamber 70 which branches towards the outlet duct 72 placed below and towards a connection 74 with the suction pipe of the pump, sidewise positioned.

The chamber 70 is provided with a diagramm 76 having a discharging opening 78, by which the same chamber 70 is put into communication with the discharging duct 72, having a plug 80 which can be controlled by means of its stem 82 , guided by a guiding system 84, controlled by a ratchet mechanism 86 having a lever 88 and a manouevering bar 90 movable in two opposite directions indicated by a double arrow 92.

A connection 94, which can be connected to the overflow pipe 18 of the overflow 16 of the tub (see figure 2), is also opening within the discharging duct 72.

The connection 74 with the suction pipe 22 is provided at its beginning part with an opening 96 closed by a valve disk 98 actuated through a stem 100 by an electromagnet actuator 102 fastened to a support 104 passing through the same connection 74.

In order to permit a controlled outflow of disinfecting solution, a connection duct 106 is used between the connection 74 and the discharging duct 72, said duct 106 comprising a first throttling element 108 of reduced diameter with an adjacently positioned movable element of valve , such as a sphere 110, maintained at a short distance or in a slight contact with the said throttling element 108 by a spring 112 abutting against a support 114 which can be spaced in adujstable manner with respect to the first throttling element 108.

The operation of the first embodiment of the present invention is as follows:

when it is desired to carry out disinfection of the hydraulic circuit 110 the outlet nozzles of the delivery fittings 30a-f are closed by placing plugs or according to a more practical alternative, delivery fittings are used which remain in a closed condition when the circulation pump 24 is not operated.

The water is fed from the duct 42, connected to the tap water system , or a disinfecting solution is fed if said duct is connected to a tank for this solution by actuating the electrovalve 44.

If water is taken from the tap water system , said water is passed through a filter 42 storing a reserve of a disinfectant for the dosage thereof and then passed in a duct 48. If, on the contrary, a disinfecting solution is used fed from a tank it is immediately fed to the said duct 48 which in any case leads the disinfecting solution to the header 27 connected to the delivery pipe 26 of the pump 24 and to the duct 28 of delivery to the nozzles 30 a, 30f, filling with disinfecting solution all these parts.

The solution is then passed through the pump 24 and then fills the whole suction pipe 22 up to the bottom valve 50 of the suction fitting 20 and starts outflowing through the duct 52 provided with throttling means connected to the discharge duct 19.

The duct 52 provided with the throttling means, being wholly similar to the duct 106 of figure 3, permits an outflow of disinfecting solution with extends in time after the closing of electrovalve 44 so as to ensure a dwell time of the solution itself in the hydraulic circuit 10 for a time sufficient to ensure the disinfection thereof.

The operation of the second embodiment of the present invention for the disinfection of the hydraulic circuit 10a is like that of the first embodiment, with the exception that in this case the disinfecting solution, after having filled the suction duct 22, stops against the valve disk 98 and starts outflowing through the duct 106 it being slowed down by the throttling element 108 and by the presence of the valve consisting of the sphere 110 and of the spring 112, so that said solution remains in the hydraulic circuit 10a for a time sufficient to ensure the disinfection thereof.

The above description relates to some embodiments given only for exemplifying purposeand not as a limitation, whereby variations and substitutions fully or partially equivalent to the above described solution shall appear to the skilled in the art, entirely falling within the scope of the present invention.

In this connection it is self-evident that the present invention is applicable as well to the cleaning of the hydromassage system by using a detergent solution instead of the disinfectant solution.

## Claims

1. Disinfection assembly of the hydraulic circuit (10,10a) of a hydromassage system for a bath tub (12), of the type comprising a circulation pump (24) at least a delivery fitting (30a-f) which can be closed , at least a suction fitting (20, 74) which can be closed, said tub (12) furthermore comprising a conventional outlet (14, 14a) characterized in that said hydraulic circuit (10, 10a) is connected through at least a controllable valve (44) to a source of disinfecting liquid (40) and in that said hydraulic circuit (10, 10a) is connected at its lowest point to the outlet of the tub through a duct (52, 106) of a diameter definitely lesser than that forming said hydraulic circuit (10,10a), is said duct a non return or check valve being mounted, which can be opened under the action of a liquid column contained in said hydraulic circuit so as to permit to outflowing of said liquid within a predetermined time such as to permit a sufficient disinfecting action in said hydraulic circuit.

2. Disinfection assembly, according to claim 1, characterized in that the outlet (14a) of the tub (12) comprises a suction fitting (74) for the hydromassage system, provided with an on-off valve (98) permitting the passage of liquid only from said outlet (14a) towards said system (10a), placed upstream of the closing means of said outlet (80), and a duct (106) provided with a throttling section (108) and with a non return valve (110), which can be opened under the action of a liquid column contained in said hydraulic circuit and connnecting a part of the suction fitting (22) downstream of its non return valve (98) with a part (72) downstream of said closing means (80) of said outlet.

3. Disinfection assembly, according to claim 2, characterized in that said on-off valve (98) on the suction fitting (14a) is a non return valve which is maintained in the closed condition by elastic means and is opened by the operation of the pump (24), of the hydromassage system generating a the depression in said suction fitting (22).

4. Disinfection assembly, according to claim 2, characterized in that said on-off valve (98) on the suction fitting is an electrovalve actuated by electromagnet (102) connected together the pump (24) of the hydromassage system.

5. Disinfection assembly according to claim 2, characterized in said said duct (106) connecting the suction pump downstream of its check valve (98) with the outlet (72) downstream of closing means (80) contains a valve (110) provided with an adjustable spring (122) to control the outflowing time of liquid from said hydromassage system.

6. Disinfection assembly, according to claims 1 and 2, characterized in that said source of disinfecting liquid (40) consist of a tank containing a disinfecting solution .

7. Disinfection assembly according to claims 1 and 2, characterized in that said source of disinfecting liquid (40) consist of a disinfectant delivery device , connected to tap water system under pressure (42) comprising a controllable valve (44), a storage of disinfecting sustance and a dosing device (46) by which the disinfecting solution is fed into said hydromassage system.

8. Assembly according to claim 1, characterized in that the treating solution in a detergent solution.

EP 0 312 953 A2

Fig.1

Fig.2

Fig.3